# EUROPEAN PATENT SPECIFICATION

(11) **EP 0 920 531 B1**
(45) Date of publication and mention of the grant of the patent: **08.09.2004**
(21) Application number: 97928067.4
(22) Date of filing: 07.07.1997
(51) Int. Cl.: C12Q 1/58

(54) **TEST STRIP FOR DETECTING GASTRIC PROBLEMS BASED ON THE PRESENCE OF UREASE**
TESTSTREIFEN ZUM NACHWEIS VON MAGENPROBLEMEN BASIEREND AUF DER ANWESENHEIT VON UREASE
BANDELETTE REACTIVE POUR DETECTER DES PROBLEMES GASTRIQUES SUR LA BASE DE LA PRESENCE D'UREASE

(30) Priority: 15.07.1996 AU 6050696
(43) Date of publication of application: 09.06.1999
(73) Proprietor: Gastro Services Pty Limited, Five Dock, New South Wales 2046 (AU)
(72) Inventor: BORODY, Thomas, Julius, Mortlake, NSW 2137 (AU); SHORTIS, Nicolas, Peter, Dural, NSW 2158 (AU)
(74) Representative: Maury, Richard Philip
(86) International application number: PCT/AU1997/000432
(87) International publication number: WO 1998/002572

(56) References cited:
- EP-A- 0 112 077
- EP-A- 0 226 767
- EP-A- 0 322 669
- EP-A- 0 369 292
- WO-A-94/13830
- US-A- 4 748 113
- US-A- 5 314 804
- US-A- 5 420 016
- US-A- 5 439 801
- US-A- 5 593 851
- DERWENT ABSTRACT, Accession No. 1990-207404 Class BO4, D16, XP002945347; & SU,A,1 507 797, (KISH MEDICAL INST), 15 September 1989
- JOURNAL OF CLINICAL MICROBIOLOGY, (Jan. 1980), Vol. 11, No. 1, EDBERG et al., "Rapid Biochemical Characterisation of Haemophilus Species by Using the Micro-ID", pages 22-26, XP002945367
- CANADIAN JOURNAL OF MEDICAL TECHNOLOGY, Vol. 45, No. 2, (1983), WOLFE J., "A Comparison of Three Methods for the Detection of Urease Activity in Mycobacteria", pages 103-109, XP002945371

## Description

### TECHNICAL FIELD

The present invention relates to a testing apparatus or diagnostic device suitable for use in diagnosing disorders in humans or lower mammal subjects. In particular the present invention is directed towards a testing apparatus suitable for diagnosis of gastrointestinal disorders by detection of the urease enzyme.

### BACKGROUND

Helicobacter pylori (H. pylori) has been implicated in causing chronic histological gastritis. Its causal role in peptic ulceration is also apparent, but less clear in relation to non-ulcer dyspepsia. The role of H. pylori can be more effectively controlled if a quick, inexpensive testing apparatus for detecting it is used. Upon detection or exclusion of H. pylori, therapeutic options can be effected to eradicate the infection or treat the symptoms if the infection is excluded.

As with the management of any disorder, the rapid, precise, and accurate diagnosis of gastrointestinal disorders is of paramount importance. However, the diagnostic methods typically employed in the art, such as histopathology, are often slow, cumbersome, costly and may yield equivocal or inaccurate results.

Helicobacter pylori can be detected by blood test for antibodies. However, the blood test can remain positive for many months after the bacteria have been eradicated so that checking a patient's success in eradicating the bacteria is not possible. Furthermore, the presence of antibodies presents a falsely positive result in approximately 10 to 15% of patients due to cross reaction with other antibodies.

Other methods for detecting H. Pylori include the ¹³C and ¹⁴C breath tests. These tests require the availability of the expensive isotopes ¹³C and ¹⁴C together with the expertise to carry out the test and the ability to detect the presence of ¹³C or ¹⁴C using very expensive equipment. This includes gas chromatography or a scintillation counter - both are expensive pieces of equipment. Hence, the direct detection of active Helicobacter pylori infection to date has been difficult to achieve unless one can obtain tissue from the stomach.

It is known that gastrointestinal disorders of the upper gastrointestinal tract may be detected and diagnosed by methods and compositions for the detection of urease enzyme in the gastric mucosa or gastric fluid of humans or lower animals. US Patent 4,748,113 (*Marshall*) describes compositions for diagnosis of gastrointestinal disorders comprising urea, a bactericide, an indicator having a pKₐ of from about 6.5 to about 8.5, and water, wherein the composition has a pH of from about 5.0 to about 6.5 and wherein the pH is at least about one pH unit lower than the pKₐ of the indicator. The compositions contain a gelling agent, such as agar and a buffer. *Marshall* teaches a method for diagnosis of gastrointestinal disorders in a human or lower animal subject, comprising the steps of obtaining a sample of gastric material from the subject, contacting the sample with a composition of this invention, and observing any color change, wherein a color change indicates the presence of a gastrointestinal disorder.. However, a problem with the composition disclosed in *Marshall* is that this test kit is bulky and requires storage at 2-8°C.

SU 1507797 (*Kish Medical Inst*.) discloses a paper sheet coated with reagent comprising (in %wt): 0.9-1.1 urea, 1.5-2.5 glucose, 0.4-0.6 polyvinyl alcohol, 0.04-0.06 bromo-thymol blue and the balance physiological solution buffered to pH 5.4-5.6.

EP 112077 (*Oxiod Limited*) discloses A diagnostic probe, for testing for the presence in a sample of an enzyme, comprises a handle and a tip, which tip is shaped to be applied to the sample and carries a reagent to react with the enzyme and thereby generate a colour signal. The probe may be a rod or stick of rolled paper or open-pore plastics material. The sample may be a bacterial colony growing on a nutrient gel. The test takes seconds, or at most minutes, and does not involve incubation.

A further test method and kit known as PYLORITEK™ described and claimed in US Patent Nos 5,314,804 and 5,420,016. utilises a number of components, such as a test pad incorporating a diffusion element, a reaction chamber and hydration solution. This test kit is also bulky, requires storage at 2 - 7°C and not simple to use.

The bulkiness and necessity to refrigerate the above mentioned kits adds expense to the storage and shipping of such kits.

It has been found that the aforesaid limitations of the known kits have imposed limitations on the extent of use of such kits, particularly in environments where storage and shipping is a problem, and an object of the invention is to alleviate that situation.

### SUMMARY OF INVENTION

The present invention overcomes the disadvantage associated with the necessary refrigeration of the prior art testing kits, by providing a testing apparatus having a dry indicating composition responsive to urease. The present invention resides in the appreciation that a dry indicating composition responsive to urease may be used, and that the moisture content required for the test to take place may be provided by the gastric material sample being tested.

In a first aspect the present invention consists of a testing apparatus for detecting urease in a gastric material taken from a mammal subject, said apparatus comprising:
a backing sheet,
a cover sheet sealingly adhered about its periphery to said backing sheet,
characterised by:
a dry indicating composition being absorbed or impregnated into or applied to said backing sheet (2) or absorbed or impregnated into or applied to a wafer carried by the backing sheet, said dry indicating composition containing urea such that it is responsive to said urease and a buffer in sufficient concentration such that in use it prevents substantial changes in pH due to chemicals other than urease in the gastric material taken from the mammal subject,
wherein said cover sheet is sealingly adhered about its periphery to said backing sheet to enclose said dry indicating composition,
wherein at least one of said backing sheet or said cover sheet has a transparent portion in alignment with said dry indicating composition,
wherein said cover sheet may be peeled from said backing sheet and resealed therewith, and
wherein said testing apparatus does not require refrigeration during storage.

Preferably the dry indicating composition prior to drying comprises a liquid composition of:
a) urea;
b) an indicator having a pKₐ, of from 6.5 to 8.5 at a concentration of 2 to 100 milligrams per litre; and
said liquid composition has a pH from 5.0 to 6.5, being at least about one pH unit lower than the pK, of said indicator.

Preferably said indicator is present at a concentration of 2 to 100 milligrams per litre.

Preferably said indicator is one of p-nitrophenol, bromothymol blue, phenol-red, neutral red, quinoline blue, cresol purple and thymol blue.

In a second aspect the present invention resides in use of the testing apparatus as defined in the first aspect above, for detection of a gastrointestinal disorder in a mammal subject by detection of urease in a gastric material sample taken from the mammal subject, comprising the steps of:
a) contacting said sample with the dry indicating composition of the testing apparatus; and
b) observing the colour of said composition; and
wherein a change of colour of said composition indicates the presence of urease and the existence of a gastrointestinal disorder in the subject.

In a third aspect of the invention, the invention resides in use of a dry indicating composition for detection of a gastrointestinal disorder in a mammal subject by detection of urease in a gastric material sample taken from the mammal subject, comprising the steps of:
a) contacting said sample with said dry indicating composition being absorbed or impregnated into or applied to said backing sheet (2) or absorbed or impregnated into or applied to a wafer carried by the backing sheet, the dry indicating composition prior to drying comprising:
   (i) urea;
   (ii) an indicator having a pKₐ of from 6.5 to 8.5 at a concentration of 2 to 100 milligrams per litre; and
   (iii) a buffer in sufficient concentration such that in use it prevents substantial changes in pH due to chemicals other than urease in the gastric material taken from the mammal subject,
said composition having a pH from 5.0 to 6.5, the pH of said composition is at least one pH unit lower than the pKₐ, of said indicator; and
wherein a change of colour of said composition indicates the presence of urease and the existence of a gastrointestinal disorder in the subject; and
wherein said testing apparatus does not require refrigeration during storage.

### BRIEF DESCRIPTION OF THE DRAWINGS

Figure 1 is a perspective view of a first embodiment of the testing apparatus of the present invention in an open configuration.
Figure 2 is a plan view of the testing apparatus shown in Figure 1 in a closed configuration.
Figure 3 is a perspective view of a second embodiment of the testing apparatus of the present invention in an open configuration.
Figure 4 is an elevational view of the second embodiment of the testing apparatus in a closed configuration.

### MODE OF CARRYING OUT INVENTION

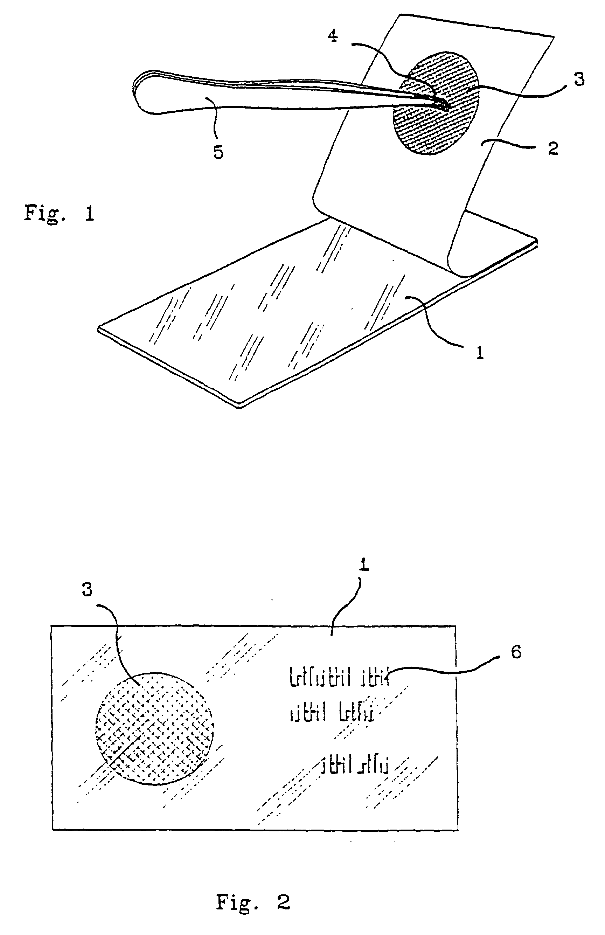

In a first embodiment of the present invention Figure 1 shows a testing apparatus in an open configuration. The testing apparatus comprises a transparent cover sheet 1 and a backing sheet 2 supporting a wafer 3 carrying a dry indicating composition. The cover sheet 1 and backing sheet 2 are joined at one end and movable with respect to each other such that wafer 3 may be sandwiched therebetween.

Preferably cover sheet 1 and backing sheet 2 are flexible water impermeable plastic strips. Suitable preferred sizes for such plastic strips are in the range of areas between 40 square millimetres and 400 square millimetres.

Wafer 3 is adhered to backing sheet 2 and is preferably made of paper or some other suitable material capable of carrying the dry indicating composition by absorption of or impregnation by an initially liquid composition which is then allowed or caused to dry out during manufacture prior to storage and use.

One or both of cover sheet 1 and backing sheet 2 have adhesive on their surfaces which meet when closed together, of the type which remains functionally tacky and allows for repeated openings and closings.

One or both of carrier sheet 1 and backing sheet 2 may carry printed matter 6, such as instructions or other labelling.

Prior to use the testing apparatus is supplied in a closed configuration, such that the dry indicating composition remains sealed and therefore uncontaminated during storage and shipping.

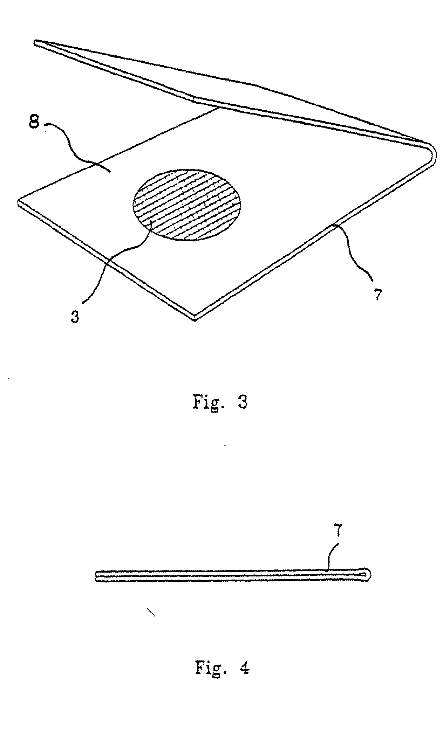

In a second embodiment as shown in Figures 3 and 4, a single sheet 7 may replace the cover and backing sheets of the first embodiment. In such an embodiment single sheet 7 supports wafer 3 and is folded in a manner to provide the function of the cover and backing sheets of the first embodiment and is also similarly provided with an adhesive on surface 8 which remains functionally tacky and allows for repeated openings and closings of folded sheet 7.

Whilst both first and second embodiments are described with reference to sheets made of plastic strip, it should be understood that other materials may be used in combination with plastic strip. For instance, backing sheet 2 may be paper or some other fibrous material capable of supporting wafer 3. Where paper or fibrous material is used for backing sheet 2, the wafer may be eliminated, and the dry indicating composition may be directly carried on backing sheet 2 by absorption or impregnation. The dry indicating composition whether carried by a wafer or directly by the backing sheet may preferably be between 5.0 to 15.0 millimetres in diameter.

The testing apparatus of the present invention is suitable for testing for the presence of urease, in order to diagnose gastrointestinal disorders of human or lower mammal subjects. As used herein, "gastrointestinal disorder" encompasses any disease or other disorder of the gastrointestinal tract of a human or lower mammal. Such gastrointestinal disorders include, for example: disorders not manifested by presence of ulcerations in the gastric mucosa (herein "no-ulcerative gastrointestinal disorder"), including chronic or atrophic gastritis, gastroenteritis, non-ulcer dyspepsia, oesophageal reflux disease and gastric motility disorder; and "peptic ulcer disease" ie., gastric and duodenal ulcers. In particular, "gastrointestinal disorder" refers to such disorders of the upper gastrointestinal tract caused or mediated by bacteria, including *Helicobacter*-like organisms, eg., *Helicobacter pylori*. Such *Helicobacter*-like organisms include those described in J. R.Warren and B. J. Marshall, "Unidentified Curved Bacilli on Gastric Epithelium of *Campylobacter-like* Bacteria that are Distinct from *'Campylobacter jeiluni'*", The Lancet 111-112(1985).

Testing is achieved by placing a sample of gastric mucosa 4 (or gastric fluid), by means for example using tweezers (or forceps) 5, against the dry indicating composition, closing the sheets together and observing a colour change in the dry indicating composition . The transparent nature of cover sheet 1 of the first embodiment or sheet 7 of the second embodiment allows for observation of colour change of the indicating composition, whilst keeping the test apparatus closed.

The test apparatus may be used to test for the presence of urease where the dry indicating composition comprises of urea and an indicator. Urea is of the formula H₂NCONH₂, and is a naturally occurring product of protein metabolism. Urea for use in the dry indicating composition, is available from a variety of commercial sources. It is known that gastric materials from humans or other animals having gastrointestinal disorders contain relatively large quantities of urease (urea amidohydrolase), which hydrolyses urea to ammonium carbonate, or ammonia and carbon dioxide. The components of the dry indicating composition serve, in part, to detect the presence of urease through its hydrolysis of urea.

In a preferred embodiment, the initially wet indicating composition prior to "drying" comprises of urea from 10 to 40 grams per litre, more preferably from 20 to 40 grams per litre, and an indicator having a pie of from 6.5 to 8.5, at an "effective concentration". The composition should have a pH from 5.0 to 6.5, and the pH of the composition is at least one pH unit lower than the pKₐ of the indicator. As used herein, "an effective concentration" of indicator is a concentration of indicator at a level from 2 to 100 milligrams per litre, which effects a readily discernible colour of the composition.

The indicators useful in this invention are weak acids, with sharply different colours in their dissociated (ionised) and undissociated (neutral) states. The indicators useful herein have pKₐ values of from 6.5 to 8.5, but more preferably from 7.0 to 8.0. The colour exhibited by the indicator in the present composition will depend on the pH of the composition, the particular indicator used, and the dissociation constant (Kₐ) for that indicator (i.e., pKₐ = log₁₀Kₐ). As the colour exhibited by the indicator changes over a range of pH values (pH = log₁₀[H⁺]), the indicators useful in the present composition change colour over a pH range of from 5.5 to 9.0, preferably from 6.5 to 8.5. The pH of the present compositions are, accordingly, adjusted to a pH at least one pH unit lower than the pKₐ of the indicator used (ie., having a hydrogen ion concentration [H⁺] ten times less than (10% of) the hydrogen ion concentration in a solution having a pH equal to the pKₐ of the indicator). Preferably, the pH is adjusted to a pH about two pH units below the pKₐ of the indicator. Adjustment of the pH of the present compositions can be effected by addition of a base (eg., sodium hydroxide) or an acid (eg., hydrochloric or citric acid). Thus, preferably, the pH of the composition of this invention is adjusted to a pH of from 5.0 to 6.5, more preferably from 5.0 to 6.0.

Indicators among those useful herein include p-nitrophenol, bromothymol blue, phenol red, neutral red, quinoline blue, cresol purple, and thymol blue. Bromothymol blue, phenol red, neutral red and cresol red are preferred indicators for use in the compositions of this invention. Indicators among those useful herein are described in The Merck Index (9th ed. 1976), incorporated by reference herein.

When manufacturing the embodiments of the earlier described test apparatus, the method of "drying" the indicating composition used can be of the order of 30°C to 80°C. Warm enough so that the paper or other material used in manufacturing the wafer 3 and/or backing sheet 2 is able to dry within a short period of time, yet cool enough so that it does not combust.

The indicating composition may contain additional optional components which affect the performance or physical characteristics of the composition. Such additional components must not, however, interfere with the indicator, ie should not obscure the colours exhibited by the indicator.

A particularly preferred optional component of the present invention is a buffer. As stated earlier, the initially wet indicating composition is adjusted to a pH at least one pH unit below the pKₐ of the indicator used. Thus, preferably, the pH of the present composition is from 5.0 to 6.5, more preferably from 5.0 to 6.0. This pH of the final composition is preferably effected by the addition of a suitable buffer.

Such buffers are well known in the chemical art, including the use of such weak acid salts as a sodium bisulfate, sodium acetate and sodium phosphate.

The total amount of buffer incorporated in the indicating composition will depend upon the total amount of urea incorporated in the composition, such that the buffer does not prevent sufficient change in composition pH (resulting from hydrolysis of the urea present) so as to cause a change in the colour of the indicator used. However, the buffer is preferably present in a concentration sufficient to prevent substantial changes in composition pH, and (hereby) spurious indicator colour changes, due to chemicals other than urease enzyme in the gastric material sample to be analysed. Typically, then, the buffer is incorporated in the present composition of concentrations of from 50 to 2000 milligrams per litre. As used herein, the buffer concentration includes the concentration of the buffer salt and of the acid used to effect pH adjustment of the composition.

Bactericide may also be optionally incorporated in the indicating composition. The bactericide may be one or more materials which substantially inhibit the growth of urease-producing organisms in the indicating composition. Suitable bactericides include sodium azide and methylhydroxybenzoate. Methylhydroxybenzoate is a particularly preferred bactericide. The specific amount of bactericide to be used depends upon factors well known in the microbiological arts, such as the particular bactericide used, and the bactericidal properties (if any) of the other components in the present compositions.

A gelling agent may also be optionally incorporated in the initially wet indicating composition, preferably in the concentration of between 5 to 50 grams per litre.

As used herein, "gastric material" refers to any moist material obtained directly or indirectly from the upper gastrointestinal tract of a human or other mammal. Such materials include, for example, gastric epithelium, gastric mucosa, and digestive fluids. Samples of such materials, for the use in the method of this invention, may be obtained by a variety of well known methods, according to sound medical practice. Such methods include, for example, obtaining the sample by biopsy of the subject, obtaining the sample from the vomitus of the subject, and obtaining the sample from nasal gastric aspirate.

As used herein, "contacting" the sample with the composition of the present invention, as in step (b) of the method set forth above, refers to any method which effects substantial interface between a dry indicating composition (of the type described earlier to detect urease), and the sample gastric material, for a time sufficiency long so as to allow the hydrolysis of urea by any urease present in the sample. Such time is typically longer than about five minutes. Also, preferably, care is taken so as to avoid contamination of the gastric material sample with organisms from a source other than the stomach of the subject to be diagnosed.

In the event that the gastric material used constitutes digestive fluids, then a preferred optional step in the present method is testing the pH of said gastric material. Preferably, then, the sample is contacted with a pH-test composition which is an aqueous solution of the particular indicator used in said composition of this invention (without urea), adjusted to the same pH as said urea-containing dry indicating composition. If the gastric material effects no change in the indicator colour of the pH-test composition, then the material is of an acid pH, and may be used directly in the contacting step of the process of this invention described above. If, however, the colour of the pH-test composition changes, then the pH of the gastric material must be acidified, as by addition of an acid.

The observing step of this method entails detection of any colour change in the composition colour, to the colour exhibited by the indicator in its dissociation state. Failure of the composition to change colour after about twenty-four hours reflects a negative test result.

The following non-limiting examples illustrate the compositions, methods and apparatus of the present invention.

### EXAMPLE I

A composition, according to this invention, was made comprising the following components:

| **Component** | **Quantity (grams)** | Final **Concentration** |
|---|---|---|
| urea | 3.000 | 30 g/l |
| phenol red | 0.008 | 80 mg/l |
| methyl hydroxy benzoate | 0.200 | 2 g/l |
| citric acid | 0.040 | 400 mg/l |
| sodium phosphate | 0.080 | 800 mg/l |

The components, except urea, were dissolved in 100 millilitres of water, heated to approximately 65° C, and stirred until the solution was clear. The solution was then cooled to below approximately 45°C, the urea added and stirred, and the solution pH was measured to be 6.0. The composition was then poured onto paper, a device of this invention, and the paper placed in an incubator set at 40°C. The composition on the paper was allowed to dry, forming a dry indicating composition on the paper having a deep yellow colour. The paper was then cut into circles of 10 millimetre diameter.

A dry indicating composition made as above is used in a method of this invention, by obtaining a sample of gastric mucosa from the stomach of a human subject presenting symptoms of gastritis. The mucosa sample is then placed on the dry indicating composition and the colour of the test paper observed. After approximately 15 minutes, a red colour is observed to completely cover the 10 millimetre diameter circle, indicating the presence of *Helicobacter*-mediated gastrointestinal disorder.

### EXAMPLE II

A composition is made, according to the present invention, comprising the following components:

| **Component** | **Quantity (grams)** | **Final Concentration** |
|---|---|---|
| urea | 2.000 | 20 g/l |
| phenol red | 0.006 | 60 mg/l |

The components were dissolved in 100ml water, and the pH adjusted to pH 6.2.

Approximately 0.5 millilitres of the composition is poured onto paper and is dried in an incubator thus forming a dry indicating composition absorbed into the paper. The dried treated paper was then cut into circles of 15 millimetres diameter as indicated in the preferred embodiments the test apparatus earlier described.

A sample of vomitus from a human infant subject suspected of having gastritis, is drawn into a syringe, and a portion injected into a pH-test composition comprising 0.6 milligrams of phenol red in 10 ml of water, adjusted to pH 6.2. the colour of the pH-test composition does not change. Thereafter, the remainder of the vomitus sample is injected onto the paper containing the dry indicating composition. The colour is observed for approximately 20 minutes, noting a change in colour from deep yellow to red, and indicating the presence of gastrointestinal disorder.

## Claims

1. A testing apparatus for detecting urease in a gastric material taken from a mammal subject, said apparatus comprising:
a backing sheet (2),
a cover sheet (1) sealingly adhered about its periphery to said backing sheet (2),
**characterised by**:
a dry indicating composition being absorbed or impregnated into or applied to said backing sheet (2) or absorbed or impregnated into or applied to a wafer carried by the backing sheet, said dry indicating composition containing urea such that it is responsive to said urease and a buffer in sufficient concentration such that in use it prevents substantial changes in pH due to chemicals other than urease in the gastric material taken from the mammal subject,
wherein said cover sheet (1) is sealingly adhered about its periphery to said backing sheet (2) to enclose said dry indicating composition,
wherein at least one of said backing sheet (2) or said cover sheet (1) has a transparent portion in alignment with said dry indicating composition,
wherein said cover sheet (1) may be peeled from said backing sheet (2) and resealed therewith, and
wherein said testing apparatus does not require refrigeration during storage.

2. A testing apparatus as claimed in claim 1, wherein said dry indicating composition, prior to drying comprises a liquid composition of:
a) urea;
b) an indicator having a pKₐ, of from 6.5 to 8.5 at a concentration of 2 to 100 milligrams per litre; and
said liquid composition has a pH from 5.0 to 6.5 being at least one pH unit lower than the pKₐ of said indicator.

3. A testing apparatus as claimed in claim 2, wherein said indicator is one of p-nitrophenol, bromothymol blue, phenol-red, neutral red, quinoline blue, cresol purple and thymol blue.

4. A testing apparatus as claimed in claim 2, wherein said urea is present at a concentration of 10 to 40 grams per litre.

5. A testing apparatus as claimed in claim 2, wherein said composition comprises a buffer.

6. Use of a testing apparatus as claimed in claim 1 for detection of a gastrointestinal disorder in a mammal subject by detection of urease in a gastric material sample taken from the mammal subject, comprising the steps of:
a) contacting said sample with the dry indicating composition of the testing apparatus; and
b) observing the colour of said composition;
wherein a change of colour of said composition indicates the presence of urease and the existence of a gastrointestinal disorder in the subject.

7. Use of a dry indicating composition in a testing apparatus for detection of a gastrointestinal disorder in a mammal subject by detection of urease in a gastric material sample taken from the mammal subject, comprising the steps of:
a) contacting said sample with said dry indicating composition being absorbed or impregnated into or applied to said backing sheet (2) or absorbed or impregnated into or applied to a wafer carried by the backing sheet, the dry indicating composition prior to drying comprising:
(i) urea;
(ii) an indicator having a pKₐ of from 6.5 to 8.5 at a concentration of 2 to 100 milligrams per litre; and
(iii) a buffer in sufficient concentration such that in use it prevents substantial changes in pH due to chemicals other than urease in the gastric material taken from the mammal subject,
said composition having a pH from 5.0 to 6.5, the pH of said composition is at least one pH unit lower than the pKₐ, of said indicator; and
b) observing the colour of said composition;
wherein a change of colour of said composition indicates the presence of urease and the existence of a gastrointestinal disorder in the subject and
wherein said testing apparatus does not require refrigeration during storage.

## Patentansprüche

1. Testvorrichtung zum Nachweisen von Urease in einem Magenmaterial, das einem Säugerobjekt entnommen wurde, umfassend:
eine Trägerfolie (2),
eine Deckfolie (1), die über ihren Umfang dichtend an die Trägerfolie (2) angeklebt ist,
**gekennzeichnet durch**:
ein trockene Indikatorzusammensetzung, die in der Trägerfolie (2) absorbiert oder imprägniert oder darauf aufgebracht ist oder die in einem Blättchen, das von der Trägerfolie getragen wird, absorbiert oder imprägniert oder darauf aufgebracht ist, wobei die trockene Indikatorzusammensetzung Harnstoff enthält, so daß sie auf die Urease anspricht, und einen Puffer in hinreichender Konzentration, so daß sie bei Gebrauch erhebliche pH-Änderungen aufgrund anderer Chemikalien als Urease in dem dem Säugerobjekt entnommenen Magenmaterial verhindert,
wobei die Deckfolie (1) über ihren Umfang dichtend an die Trägerfolie (2) angeklebt ist, um die trockene Indikatorzusammensetzung einzuschließen,
wobei wenigstens entweder die Trägerfolie (2) oder die Deckfolie (1) einen durchsichtigen Teil aufweist, der gemäß der trockenen Indikatorzusammensetzung ausgerichtet ist,
wobei die Deckfolie (1) von der Trägerfolie (2) abgezogen und damit wieder verschlossen werden kann, und
wobei die Testvorrichtung keine Kühlung während der Lagerung benötigt.

2. Testvorrichtung nach Anspruch 1, wobei die trockene Indikatorzusammensetzung vor dem Trocknen eine flüssige Zusammensetzung aus:
a) Harnstoff
b) einem Indikator mit einem pKₐ von 6,5 bis 8,5 in einer Konzentration von 2 bis 100 Milligramm pro Liter umfaßt,
und die flüssige Zusammensetzung einen pH von 5,0 bis 6,5 aufweist und somit wenigstens eine pH-Einheit niedriger liegt als der pKₐ des Indikators.

3. Testvorrichtung nach Anspruch 2, wobei der Indikator einer aus p-Nitrophenol, Bromthymolblau, Phenolrot, Neutralrot, Chinolinblau, Cresolpurpur und Thymolblau ist.

4. Testvorrichtung nach Anspruch 2, wobei der Harnstoff in einer Konzentration von 10 bis 40 Gramm pro Liter vorhanden ist.

5. Testvorrichtung nach Anspruch 2, wobei die Zusammensetzung einen Puffer umfaßt.

6. Verwendung einer Testvorrichtung nach Anspruch 1 zum Nachweisen einer gastrointestinalen Störung in einem Säugerobjekt durch Nachweisen von Urease in einem Magenmaterial, das dem Säugerobjekt entnommen wurde, umfassend die Schritte:
a) Zusammenbringen der Probe mit der trockenen Indikatorzusammensetzung der Testvorrichtung, und
b) Beobachten der Farbe der Zusammensetzung,
wobei eine Farbänderung der Zusammensetzung das Vorhandensein von Urease und das Vorliegen einer gastrointestinalen Störung im Objekt anzeigt.

7. Verwendung einer trockenen Indikatorzusammensetzung in einer Testvorrichtung zum Nachweisen einer gastrointestinalen Störung in einem Säugerobjekt durch Nachweisen von Urease in einem Magenmaterial, das dem Säugerobjekt entnommen wurde, umfassend die Schritte:
a) Zusammenbringen der Probe mit der trockenen Indikatorzusammensetzung, die in der Trägerfolie (2) absorbiert oder imprägniert oder darauf aufgebracht ist oder die in einem Blättchen, das von der Trägerfolie getragen wird, absorbiert oder imprägniert oder darauf aufgebracht ist, wobei die trockene Indikatorzusammensetzung vor dem Trocknen umfaßt:
(i) Harnstoff
(ii) einen Indikator mit einem pKₐ von 6,5 bis 8,5 in einer Konzentration von 2 bis 100 Milligramm pro Liter, und
(iii) einen Puffer in hinreichender Konzentration, so daß sie bei Gebrauch erhebliche pH-Änderungen aufgrund anderer Chemikalien als Urease in dem dem Säugerobjekt entnommenen Magenmaterial verhindert,
wobei die Zusammensetzung einen pH von 5,0 bis 6,5 aufweist und der pH der Zusammensetzung wenigstens eine pH-Einheit niedriger als der pKₐ des Indikators ist; und
b) Beobachten der Farbe der Zusammensetzung,
wobei eine Farbänderung der Zusammensetzung das Vorhandensein von Urease und das Vorliegen einer gastrointestinalen Störung im Objekt anzeigt, und
wobei die Testvorrichtung keine Kühlung während der Lagerung benötigt.

## Revendications

1. Appareil d'essai destiné à détecter de l'uréase dans une matière gastrique prélevée chez un sujet mammifère, ledit appareil comprenant :
une feuille de renforcement (2),
une feuille de couverture (1) collée de manière étanche aux environs de sa périphérie à ladite feuille de renforcement (2) ,
**caractérisé par** :
une composition d'indication sèche qui est absorbée ou imprégnée dans ou appliquée à ladite feuille de renforcement (2) ou absorbée ou imprégnée dans ou appliquée à une pastille portée par la feuille de renforcement, ladite composition d'indication sèche contenant de l'urée de sorte qu'elle est réactive à ladite uréase et un tampon en concentration suffisante de sorte qu'en utilisation, elle empêche des changements substantiels en pH dus à des produits chimiques autres que l'uréase dans la matière gastrique prélevée chez le sujet mammifère,
dans lequel ladite feuille de couverture (1) est collée de manière étanche aux environs de sa périphérie à ladite feuille de renforcement (2) pour enfermer ladite composition d'indication sèche,
dans lequel au moins l'une de ladite feuille de renforcement (2) ou de ladite feuille de couverture (1) a une partie transparente en alignement avec ladite composition d'indication sèche,
dans lequel ladite feuille de couverture (1) peut être arrachée de ladite feuille de renforcement (2) et rescellée avec celle-ci, et
dans lequel ledit appareil d'essai ne requiert pas de réfrigération pendant le stockage.

2. Appareil d'essai selon la revendication 1, dans lequel ladite composition d'indication sèche, avant le séchage comprend une composition liquide :
a) d'urée ;
b) d'un indicateur ayant un pKₐ de 6,5 à 8,5 à une concentration de 2 à 100 milligrammes par litre ; et
ladite composition liquide a un pH de 5,0 à 6,5 qui est d'au moins une unité de pH inférieure au pKₐ dudit indicateur.

3. Appareil d'essai selon la revendication 2, dans lequel ledit indicateur est l'un parmi le p-nitrophénol, le bleu de bromothymol, le rouge de phénol, le rouge neutre, le bleu de quinoléine, le violet de crésol et le bleu de thymol.

4. Appareil d'essai selon la revendication 2, dans lequel ladite urée est présente à une concentration de 10 à 40 grammes par litre.

5. Appareil d'essai selon la revendication 2, dans lequel ladite composition comprend un tampon.

6. Utilisation d'un appareil d'essai selon la revendication 1 pour la détection d'un trouble gastro-intestinal chez un sujet mammifère par la détection d'uréase dans un échantillon de matière gastrique prélevée chez le sujet mammifère, comprenant les étapes consistant à :
a) mettre en contact ledit échantillon avec la composition d'indication sèche de l'appareil d'essai ; et
b) observer la couleur de ladite composition ;
dans laquelle un changement de couleur de ladite composition indique la présence d'uréase et l'existence d'un trouble gastro-intestinal chez le sujet.

7. Utilisation d'une composition d'indication sèche dans un appareil d'essai pour la détection d'un trouble gastro-intestinal chez un sujet mammifère par la détection d'uréase dans un échantillon de matière gastrique prélevée chez le sujet mammifère, comprenant les étapes consistant à :
a) mettre en contact ledit échantillon avec ladite composition d'indication sèche qui est absorbée ou imprégnée dans ou appliquée à ladite feuille de renforcement (2) ou absorbée ou imprégnée dans ou appliquée à une pastille portée par la feuille de renforcement, la composition d'indication sèche avant le séchage comprenant :
(i) de l'urée ;
(ii) un indicateur ayant une pKₐ de 6,5 à 8,5 à une concentration de 2 à 100 milligrammes par litre ; et
(iii) un tampon en concentration suffisante de sorte qu'en utilisation il empêche des changements substantiels en pH dus à des produits chimiques autres que l'uréase dans la matière gastrique prélevée chez le sujet mammifère,
ladite composition ayant un pH de 5,0 à 6,5, le pH de ladite composition est d'au moins une unité de pH inférieure au pKₐ dudit indicateur ; et
b) observer la couleur de ladite composition ;
dans laquelle un changement de couleur de ladite composition indique la présence d'uréase et l'existence d'un trouble gastro-intestinal chez le sujet et
dans laquelle ledit appareil d'essai ne requiert pas de réfrigération pendant le stockage.
